# EUROPÄISCHE PATENTSCHRIFT

(11) **EP 0 769 042 B1**
(45) Veröffentlichungstag und Bekanntmachung des Hinweises auf die Patenterteilung: **03.01.2001**
(21) Anmeldenummer: 95924915.2
(22) Anmeldetag: 22.06.1995
(51) Int. Cl.: C11C 3/10, C11C 3/12, C07C 33/025, C07C 67/02, C07C 29/149, C07C 69/58, C11D 1/68

(54) **UNGESÄTTIGTE FETTSTOFFE MIT VERBESSERTEM KÄLTEVERHALTEN**
UNSATURATED FATTY SUBSTANCES WITH IMPROVED COLD BEHAVIOUR
SUBSTANCES GRASSES INSATUREES A COMPORTEMENT AU FROID AMELIORE

(30) Priorität: 30.06.1994 DE 4422858
(43) Veröffentlichungstag der Anmeldung: 23.04.1997
(73) Patentinhaber: Cognis Deutschland GmbH, 40589 Düsseldorf (DE)
(72) Erfinder: ANSMANN, Achim, D-40699 Erkrath (DE); DEMMERING, Günther, D-42653 Solingen (DE); ASSMANN, Georg, D-41363 Jüchen (DE); SCHUSTER, Fritz, D-40589 Düsseldorf (DE); WESTFECHTEL, Alfred, D-40723 Hilden (DE); KÖHLER, Michael, D-40822 Mettmann (DE)
(86) Internationale Anmeldenummer: EP9502440
(87) Internationale Veröffentlichungsnummer: WO9600768

(56) Entgegenhaltungen:
- EP-A- 0 370 273
- EP-A- 0 496 504

## Beschreibung

Die Erfindung betrifft ungesättigte Fettstoffe mit verbessertem Kälteverhalten, die man erhält, indem man Neues LS-Sonnenblumenöl mit Methanol umestert und die resultierenden Methylester unter Erhalt der Doppelbindugen zu den entsprechenden Fettalkoholen hydriert. Die Erfindung erstreckt sich ferner auf Derivate der ungesättigten Fettalkohole sowie auf Verfahren zu deren Herstellung und deren Verwendung zur Herstellung oberflächenaktiver Mittel. Ein letzter Gegenstand der Erfindung betrifft schließlich die Verwendung des Neuen LS-Sonnenblumenöls zur Herstellung ungesättigter Fettalkohole.

### Stand der Technik

Fettstoffe, insbesondere ungesättigte Fettalkohole, stellen wichtige Zwischenprodukte für eine große Anzahl von Erzeugnissen der chemischen Industrie dar, wie z.B. für die Herstellung von Tensiden und kosmetischen Produkten. Eine Übersicht zu diesem Thema findet sich beispielsweise von U.Ploog et al. in **Seifen-Öle-Fette-Wachse 109, 225 (1983).**

Die Herstellung ungesättigter Fettalkohole gelingt nicht auf Basis petrochemischer Rohstoffe und Verfahren. Man geht vielmehr von mehr oder minder ungesättigten Fettsäuren oder deren Methylestern auf Basis nachwachsender Rohstoffe aus, die beispielsweise in Gegenwart von chrom- und/oder zinkhaltigen Mischoxidkatalysatoren unter Erhalt der Doppelbindungen hydriert werden [vgl. **Ullmann's Enzyklopaedie der technischen Chemie, Verlag Chemie, Weinheim, 4. Aufl., Bd.11, S. 436f].**

Die Herstellung ungesättigter Fettalkohole kann grundsätzlich auf drei Wegen erfolgen:
1. Fette und Öle werden einer Druckspaltung mit Wasser unterworfen. Nach Abtrennung des wäßrigen Glycerins werden Spaltfettsäuren erhalten, die Gemische gesättigter und ungesättigter Fettsäuren darstellen. Da eine gemeinsame Hydrierung dieser Säuren das Verhältnis gesättigter und ungesättigter Anteile nicht beeinflußen kann, werden auf diesem Wege nur Fettalkohole einer niedrigen Iodzahl im Bereich kleiner 80, vorzugsweise 50 bis 55 erhalten.
2. Eine destillative Trennung gesättigter und ungesättigter Fettsäuren ist nur mit einem unverhältnismäßig hohen technischem Aufwand möglich. Abweichend von (1) können die Spaltfettsäuren jedoch über den Weg der "Umnetztrennung" in einen überwiegend gesättigten und einen überwiegend ungesättigten Fettsäureschnitt überführt werden. Die Hydrierung des ungesättigten Fettsäureanteils liefert technische Oleylalkohole eines Iodzahlbereiches von etwa 80 bis 85, die in der Technik durch fraktionierte Destillation bzw. Winterisierung zu Produkten mit einer Iodzahl im Bereich von 90 bis 100 weiterverarbeitet werden.
3. Weiterhin ist es möglich, hochungesättigte Pflanzenöle einer Umesterung zu unterwerfen, bei der Methylester mit einem vergleichsweise geringen Anteil an gesättigten Homologen anfallen. Eine Umnetztrennung ist in diesem Fall weder möglich, noch erforderlich, da die Hydrierung unmittelbar hochungesättigte Fettalkohole (IZ > 100) liefert.

Die drei genannten Verfahren werden seit langem kommerziell zur Herstellung von ungesättigten Fettalkoholen genutzt, die jedoch mit einer Reihe von Nachteilen verbunden sind:
*** Die nach Verfahren 1 erhältlichen Produkte besitzen eine Iodzahl unterhalb von 80 und sind wachsartig. Neben des unvorteilhaften Erstarrungspunktes weisen sie die Vorteile, die mit der ungesättigten Struktur verbunden sind, naturgemäß nur partiell auf.
*** Üblicherweise kommen als Rohstoffe für Verfahren 2 Fette und Öle mit einer Iodzahl im Bereich von 40 bis 70, wie beispielsweise Rindertalg, Schweineschmalz, Palmöl oder Palmstearin in Frage. Die resultierenden Fettalkohole weisen eine Iodzahl im Bereich von 90 bis 100 auf und kommen aufgrund ihres Eigenschaftsprofils am ehesten für eine technische Verwendung in Betracht. Sie sind jedoch oftmals sowohl bezüglich ihrer Farbe als auch der Geruchsqualität nicht zufriedenstellend und weisen für viele Anwendungen einen ebenfalls unvorteilhaft hohen Erstarrungs- bzw. Trübungspunkt auf. Letzteres trifft im übrigen auch für ungesättigte Fettalkohole des gleichen Iodzahlbereiches auf Basis von konventionellem Neuen Sonnenblumenöl zu, das aufgrund seines hohen Anteils an Ölsäure bei niedrigem Gehalt an mehrfach ungesättigten Fettsäuren als Einsatzstoff ebenfalls in Betracht kommen könnte.
*** Für die Herstellung hochungesättigter Fettalkohole nach Verfahren 3 kommen z.B. Rapsöl, Olivenöl, Leinöl oder Erdnußöl in Frage. Hochungesättigte Fettalkohole - wie beispielsweise solchen auf Basis von erucasäurearmem Neuem Rapsöl - enthalten jedoch einen signifikanten Anteil an mehrfach ungesättigten Homologen und sind damit anfällig gegenüber Autoxidationsprozessen.

Die Aufgabe der Erfindung hat somit darin bestanden, ungesättigte Fettalkohole mit einer Iodzahl im Bereich von 90 bis 100 auf Basis pflanzlicher Rohstoffe - sowie entsprechende Folgeprodukte - zur Verfügung zu stellen, die sich insbesondere durch ein verbessertes Kälteverhalten auszeichnen.

### Beschreibung der Erfindung

Gegenstand der Erfindung sind ungesättigte Fettalkohole im lodzahlbereich von 90 bis 100 mit Trübungspunkten von 1°C, dadurch erhältlich, daß man
(a) Neues LS-Sonnenblumenöl mit einem Ölsäureanteil von mehr als 85 Gew.-% und einem Stearinsäureanteil von weniger als 3 Gew.-% mit Methanol umestert und
(b) die resultierende Methylester in an sich bekannter Weise zu den entsprechenden ungesättigten Fettalkoholen im Iodzahlbereich 90 bis 100 hydriert.

Überraschenderweise wurde gefunden, daß durch den Einsatz von Neuem Sonnenblumenöl mit niedrigem Stearinsäuregehalt (LS = "low stearic") pflanzliche Fettalkohole im Iodzahlbereich von 90 bis 100 erhalten werden, die nicht nur außergewöhnlich gute Farb- und Geruchseigenschaften aufweisen, sondern sich zudem noch in gewünschter Weise durch ein besonders vorteilhaftes Kälteverhalten auszeichnen. Insbesondere weisen die erfindungsgemäßen ungesättigten Fettalkohole gegenüber technischem Oleylalkohol auf Basis "konventionellem" Neuem Sonnenblumenöl (d.h. einem Sonnenblumenöl mit ca. 80 bis 85 Gew.-% Ölsäure und mehr als 3 Gew.-% Stearinsäure) einen überraschend niedrigen Trübungspunkt von 1°C gegenüber 18°C auf.

Weitere vorteilhafte Ausgestaltungen der Erfindung bestehen ferner in Derivaten mit ebenfalls vorteilhaftem Kälteverhalten sowie verbesserten Farb- und Geruchseigenschaften, die man erhält, indem man die eingangs genannten ungesättigten Fettalkohole in an sich bekannter Weise
*** alkoxyliert;
*** alkoxyliert, sulfatiert und neutralisiert;
*** sulfatiert und neutralisiert; bzw.
*** mit aliphatischen Carbonsäuren mit 1 bis 22 Kohlenstoffatomen und 0 und/oder 1 bis 3 Doppelbindungen verestert.

### Herstellverfahren

Ein weiterer Gegenstand der Erfindung betrifft ein Verfahren zur Herstellung ungesättigter Fettstoffe mit verbessertem Kälteverhalten, bei dem man
(a) Neues LS-Sonnenblumenöl mit einem Ölsäureanteil von mehr als 85 Gew.-% und einem Stearinsäureanteil von weniger als 3 Gew.-% mit Methanol umestert und
(b) die resultierende Methylester in an sich bekannter Weise zu den entsprechenden ungesättigten Fettalkoholen im Iodzahlbereich 90 bis 100 hydriert.

### Neues LS-Sonnenblumenöl

Bei dem Neuem "low-stearic" (LS) Sonnenblumenöl handelt es sich beispielsweise um ein in kommerziellem Umfang verfügbares Pflanzenöl, das von der Pioneer Corp./USA vertrieben wird [vgl. **EP-A1 0496504, US 5276264**] und die folgende typische Zusammensetzung gemäß Tabelle 1 aufweist:

**Tabelle 1**

| Zusammensetzung Neues LS-Sonnenblumenöl | |
|---|---|
| **Fettsäurekomponenten** | **Anteil Gew.-%** |
| Palmitinsäure | 2 bis 5 |
| Stearinsäure | 0,5 bis 2 |
| Ölsäure | 85 bis 95 |
| Linolsäure | 3 bis 5 |

Ein besonderes Kennzeichen dieses neuen Pflanzenöls besteht darin, daß es mehr als 85 und vorzugsweise mehr als 90 Gew.-% Ölsäure und weniger als 3 Gew.-% Stearinsäure enthält.

### Umesterung

Unter Umesterung (Alkoholyse) versteht man den Austausch eines im Ester gebundenen Alkohols durch einen anderen. Für die Umesterung von Triglyceriden mit Methanol gilt die Reaktionsgleichung (1):

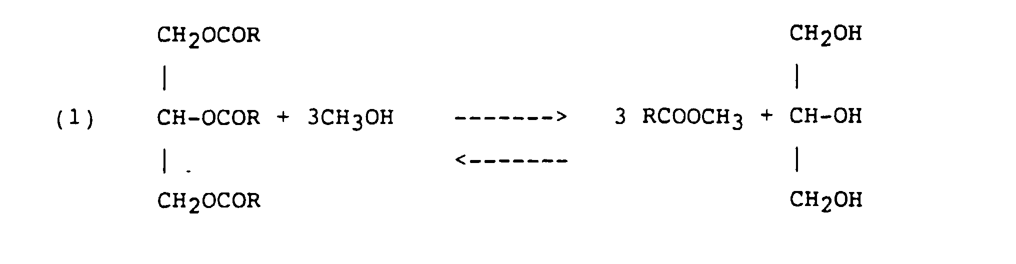

Die Umesterung stellt ebenso wie die Veresterung eine Gleichgewichtsreaktion dar. Ein Überschuß an Methanol bzw. das Abtrennen des Glycerins empfiehlt sich daher, um das Gleichgewicht der Reaktion auf die Seite der Methylester zu verschieben.

Der Austausch der Glycerinkomponente von Fetten gegen einwertige Alkohole wie Methanol ist ohne Schwierigkeiten bereits bei niedrigen Temperaturen möglich. Zur Verarbeitung werden vorzugsweise vorentsäuerte Öle und Fette eingesetzt. Diese werden bei ca. 50 bis 70°C mit einem Überschuß an reinem Methanol in Gegenwart eines alkalischen Katalysators (beispielsweise einer Zinkseife) gemischt. Nach einer entsprechenden Verweilzeit scheidet sich das Glycerin praktisch wasserfrei am Boden des Reaktionsgefäßes ab. Die Umesterungsreaktion ist abgeschlossen, sobald im gebildeten Methylester kein Glycerin mehr vorhanden ist. Die Reaktionsgeschwindigkeit kann bei dieser Batch-Fahrweise selbstverständlich durch höhere Temperaturen beträchtlich gesteigert werden.

Vorzugsweise erfolgt die Umesterungsreaktion jedoch in kontinuierlicher Fahrweise entweder drucklos bei etwa 70°C oder bei Überdruck (10 bis 90 bar, 200 bis 260°C).

Die Triglyceride werden üblicherweise zusammen mit dem Methanol, das in mehrfach molarem Überschuß eingesetzt wird, mit Hilfe von Dosierpumpen im Gleichstrom dem Umesterungsreaktor zugeführt. Gleichzeitig wird die erforderliche Katalysatormenge zudosiert. Durch Entspannung gelangt das fertige Umesterungsgemisch (Methylester, Glycerin, Methanol) in eine Methanol- und Glycerinabtrennanalage, deren wesentliche Anlagenteile aus einer Bodenkolonne und einem Glycerinabscheider bestehen. Im Verstärkerteil der Kolonne wird das abgetriebene wasserhaltige Methanol aufkonzentriert. Das Reinmethanol wird kondensiert und in den Umesterungsprozeß zurückgeführt. Im Abscheider trennt sich die verbleibende Lösung in eine Methylesterphase, die in einer nachgeschalteten Destillationsstufe gereinigt wird und eine Glycerinphase (Glyceringehalt > 90 Gew.-%), die ebenfalls aufgearbeitet wird. Verfahren zur drucklosen bzw. Niederdruckumesterung sind beispielsweise in einem Artikel von Davidsohn in **Seifen-Öle- Fette-Wachse 114, 595 (1988)** sowie der **DE-A1 3932514** (Henkel) beschrieben.

### Hydrierung

Die Reduktion von Estern mit metallischem Natrium in Gegenart eines Alkohols wurde schon 1903 von Bouveault und Blanc entdeckt. Die großtechnische Herstellung von Fettalkoholen erfolgt heute jedoch praktisch ausschließlich durch Hochdruckhydrierung von destillierten bzw. fraktionierten Methylester-bzw. Fettsäureschnitten in einem oder mehreren hintereinandergeschalteten Festbett- oder Schachtreaktoren bei Temperaturen von 200 bis 250°C und einem Wasserstoffdruck von 200 bis 300 bar. Dazu werden Fettsäure oder Methylester kontinuierlich gegen den Wasserstoffdruck in die Anlage gepreßt, auf Reaktionstemperatur erhitzt und am Reaktorkopf aufgegeben. Zur Herstellung ungesättigter Fettalkohole werden üblicherweise Adkins-Katalysatorschüttungen auf Basis von Cu/Cr/Zn und/oder Cu/Cr/Cd-Mischoxiden eingesetzt; in diesem Fall kommt es zu einer selektiven Hydrierung der Carboxyl(at)-gruppe unter Erhalt der im Fettrest enthaltenen Doppelbindungen.

Neben der Festbettfahrweise kann die Hydrierung auch in der Rieselphase durchgeführt werden. Auch bei dieser Verfahrensvariante durchströmen Fettsäure bzw. Ester und Wasserstoff den Reaktor bei einer Temperatur von 200 bis 300°C und einem Druck von 250 bis 300 bar von oben. Jedoch sind hier die Kreisgasmengen und der molare Wasserstoffüberschuß erheblich geringer, was sich in kleineren Anlagendimensionen wiederspiegelt. Als Katalysatoren werden Silikagel-Trägerkontakte mit 20 bis 40 Gew.-% der eingangs genannten Cupferchromite verwendet. Diese Katalysatoren besitzen zwar eine hohe mechanische Stabilität, sind jedoch wegen ihres geringen Gehaltes an Aktivsubstanz anfälliger gegen Vergiftungen als Massivkontakte und besitzen daher kürzere Standzeiten.

Vorzugsweise werden die resultierenden Fettalkohole anschließend unter Abnahme eines Vorlaufes (etwa 5 Gew.-%) in an sich bekannter Weise destillativ gereingt.

### Derivatisierung

Wie schon eingangs geschildert, besteht eine weitere Erkenntnis der vorliegenden Erfindung darin, daß die hervorragenden Eigenschaften der primär hergestellten ungesättigten Fettalkohole auch nach Derivatisierung erhalten bleiben. Hierzu zählen:
*** **Alkoxylierung**. Alkoxylate der ungesättigten Fettalkohole werden an sich bekannter Weise durch Anlagerung von Ethylen- und/oder Propylenoxid in Gegenwart basischer Katalysatoren wie z.B. Natriummethylat oder calcinierter Hydrotalcit erhalten und können sowohl eine konventionelle als auch eine eingeengte Homologenverteilung aufweisen. Die Alkoxylate eignen sich z.B. als Waschmittelrohstoffe, Emulgatoren im Bereich der Textiltechnik, bei Bohr- und Schneidölen sowie in kosmetischen Formulierungen.
*** **Alkoxylierung/Sulfatierung**. Ethersulfate der ungesättigten Fettalkohole werden in an sich bekannter Weise durch Alkoxylierung, nachfolgende Sulfatierung mit gasförmigem Schwefeltrioxid oder Chlorsulfonsäure sowie abschließende Neutralisation mit Basen erhalten. Die Produkte eignen sich als Waschrohstoffe.
*** **Sulfatierung**. Fettalkoholsulfate auf Basis der ungesättigten Alkohole werden in an sich bekannter Weise durch Sulfatierung mit gasförmigem Schwefeltrioxid oder Chlorsulfonsäure sowie abschließende Neutralisation mit Basen erhalten. Die Produkte eignen sich ebenfalls als Waschrohstoffe und Hilfsmittel in der Textiltechnik.
*** **Veresterung**. Ester der ungesättigten Fettalkohole werden in an sich bekannter Weise durch katalytische Umsetzung mit aliphatischen Carbonsäuren mit 1 bis 22, vorzugsweise 6 bis 22 und insbesondere 12 bis 18 Kohlenstoffatomen und 0 und/oder 1 bis 3 Doppelbindungen erhalten. Typische Beispiele sind Umsetzungen eines erfindungsgemäßen technischen Oleylalkohols (Iodzahl 95) mit Essigsäure, C₆-C₁₀-Vorlauffettsäure, Laurinsäure, Palmitinsäure, Stearinsäure, Ölsäure, C_{12/14}-Kokosfettsäure, C_{12/18}-Kokosfettsäure oder C_{16/18}-Talgfettsäure. Die Produkte eignen sich beispielsweise als Ölkörper zur Herstellung kosmetischer Mittel.

### Gewerbliche Anwendbarkeit

Die erfindungsgemäßen ungesättigten Fettstoffe zeichnen sich gegenüber den Produkten des Stands der Technik durch einen verbesserten Geruch, eine verbesserte Farbe und insbesondere durch ein vorteilhafteres Kälteverhalten aus.

Ein weiterer Gegenstand der Erfindung betrifft ihre Verwendung - alleine oder in Abmischung mit bekannten ungesättigten Fettstoffen - zur Herstellung oberflächenaktiver Mittel, wie z.B. Überfettungs- oder Lösungsmitteln für Wirkstoffe, Cremes, Salben und Lotionen, Schmiermittel in der Metallbearbeitung sowie Antischaummittel in Dispersionsfarben, in denen sie in Mengen von 1 bis 75, vorzugsweise 5 bis 50 Gew.-%-bezogen auf die Mittel - enthalten sein können.

Ein weiterer Gegenstand der Erfindung betrifft schließlich die Verwendung von Neuem LS-Sonnenblumenöl mit einem Gehalt von mehr als 90 Gew.-% Ölsäure und weniger als 3 Gew.-% Stearinsäure zur Herstellung von ungesättigten Fettalkoholen im Iodzahlbereich von 90 bis 100 über den Weg der Umesterung und Hydrierung.

### Beispiele

### Allgemeine Herstellvorschriften

**Verfahren 1**. Die Einsatzstoffe wurden einer Umesterung mit Methanol unterworfen und von Glycerin und nicht umgesetztem Methanol befreit. Das resultierende Methylestergemisch wurde nach Destillation unter Erhalt der Doppelbindungen hydriert. Anschließend wurde das Produkt durch Destillation gereinigt.

**Verfahren 2**. Die Einsatzstoffe wurden einer Druckspaltung unterworfen und das Glycerin/Wasser-Gemisch abgetrennt. Das resultierene Spaltfettsäurengemisch wurde in der Umnetztrennung in einen Stearin- und einen Oleinanteil aufgetrennt. Das Olein, das etwa 70 Gew.-% Ölsäure enthielt, wurde im Anschluß unter Erhalt der Doppelbindungen hydriert. Anschließend wurde das Produkt durch Destillation gereinigt.

Die Zusammensetzung der eingesetzten Rohstoffe ist in Tabelle 2 wiedergegeben (Prozentangaben als Gew.-%). Tabelle 3 können die anwendungstechnischen Daten der resultierenden ungesättigten Fettalkohole entnommen werden.

**Tabelle 2**

| Eingesetzte Rohstoffe | | | |
|---|---|---|---|
| **Fettsäurekomponente** | **LS %** | **NS %** | **RT %** |
| Palmitinsäure | 4 | 5 | 5 |
| Stearinsäure | 1 | 4 | 2 |
| Ölsäure | 91 | 85 | 68 |
| Linolsäure | 4 | 5 | 12 |
| Legende: | | | |
| LS = Neues "low-stearic" Sonnenblumenöl, Pioneer Corp./US | | | |
| NS = Neues Sonnenblumenöl SVO Enterprises/US | | | |
| RT = Basis Rindertalg; zum besseren Vergleich ist die Zusammensetzung der nach der Umnetztrennun resultierenden technischen Ölsäure Edenor^{(R)} F-TiO₅, Henkel KGaA/FRG angegeben. | | | |

**Tabelle 3**

| Kenndaten der Produkte | | | | | | |
|---|---|---|---|---|---|---|
| **Bsp.** | **Rohstoff** | **Ungesättigter Fettalkohol** | | | | |
| | | **IZ** | **Verf.** | **TP °C** | **Farbe APHA** | **Geruch** |
| 1 | LS-NSB-öl | 93 | 1 | 1,0 | 10 | ++ |
| V1 | NSB-Öl | 88 | 1 | 18,0 | 15 | + |
| V2 | Rindertalg | 95 | 2 | 8,0 | 100 | - |
| Legende: | | | | | | |
| IZ = Iodzahl | | | | | | |
| Verf. = Verfahrenstyp | | | | | | |
| TP = Trübungspunkt | | | | | | |
| Geruch = ++ = geruchsfrei | | | | | | |
| + = leichter Geruch wahrnehmbar | | | | | | |
| - = Geruch deutlich wahrnehmbar | | | | | | |

## Patentansprüche

1. Ungesättigte Fettalkohole im lodzahlbereich von 90 bis 100 mit einem Trübungspunk von 1 °C, dadurch erhältlich, daß man
(a) Neues Sonnenblumenöl mit niedrigem Stearinsäuregehalt mit einem Ölsäureanteil von mehr als 85 Gew.-% und einem Stearinsäureanteil von weniger als 3 Gew.-% mit Methanol umestert und
(b) die resultierenden Methylester in an sich bekannter Weise zu den entsprechenden ungesättigten Fettalkoholen im lodzahlbereich 90 bis 100 hydriert.

2. Derivate der ungesättigten Fettalkohole nach Anspruch 1, **dadurch gekennzeichnet**, daß man die ungesättigten Fettalkohole anschließend in an sich bekannter Weise alkoxyliert.

3. Derivate der ungesättigten Fettalkohole nach Anspruch 1, **dadurch gekennzeichnet**, daß man die ungesättigten Fettalkohole anschließend in an sich bekannter Weise alkoxyliert, sulfatiert und neutralisiert.

4. Derivate der ungesättigten Fettalkohole nach Anspruch 1, **dadurch gekennzeichnet**, daß man die ungesättigten Fettalkohole anschließend in an sich bekannter Weise sulfatiert und neutralisiert.

5. Derivate der ungesättigten Fettalkohole nach Anspruch 1, **dadurch gekennzeichnet**, daß man die ungesättigten Fettalkohole anschließend in an sich bekannter Weise mit aliphatischen Carbonsäuren mit 1 bis 22 Kohlenstoffatomen und 0 und/oder 1 bis 3 Doppelbindungen verestert.

6. Verfahren zur Herstellung ungesättigter Fettstoffe mit verbessertem Kälteverhalten, **dadurch gekennzeichnet**, daß man
(a) Neues Sonnenblumenöl mit niedrigem Stearinsäuregehalt mit einem Ölsäureanteil von mehr als 85 Gew.-% und einem Stearinsäureanteil von weniger als 3 Gew.-% mit Methanol umestert und
(b) die resultierenden Methylester in an sich bekannter Weise zu den entsprechenden ungesättigten Fettalkoholen im lodzahlbereich 90 bis 100 hydriert.

7. Verfahren nach Anspruch 6, **dadurch gekennzeichnet**, daß man die ungesättigten Fettalkohole anschließend in an sich bekannter Weise alkoxyliert.

8. Verfahren nach Anspruch 6, **dadurch gekennzeichnet**, daß man die ungesättigten Fettalkohole anschließend in an sich bekannter Weise alkoxyliert, sulfatiert und neutralisiert.

9. Verfahren nach Anspruch 6, **dadurch gekennzeichnet**, daß man die ungesättigten Fettalkohole anschließend in an sich bekannter Weise sulfatiert und neutralisiert.

10. Verfahren nach Anspruch 6, **dadurch gekennzeichnet**, daß man die ungesättigten Fettalkohole anschließend in an sich bekannter Weise mit aliphatischen Carbonsäuren mit 1 bis 22 Kohlenstoffatomen und 0 und/oder 1 bis 3 Doppelbindungen verestert.

11. Verwendung von ungesättigten Fettalkoholen und deren Derivate nach den Ansprüchen 1 bis 5 - alleine oder in Abmischung mit bekannten ungesättigten Fettstoffen - zur Herstellung oberflächenaktiver Mittel.

12. Verwendung von Neuem Sonnenblumenöl mit niedrigem Stearinsäuregehalt mit einem Ölsäureanteil von mehr als 85 Gew.-% und einem Stearinsäureanteil von weniger als 3 Gew.-% zur Herstellung ungesättigter Fettalkohole im lodzahlbereich 90 bis 100 über den Weg der Umesterung und Hydrierung nach Anspruch 6.

## Claims

1. Unsaturated fatty compounds with improved low-temperature behavior obtainable by
(a) transesterifying new LS sunflower oil with an oleic acid content of more than 85% by weight and a stearic acid content of less than 3% by weight with methanol and
(b) hydrogenating the resulting methyl esters in known manner to form the corresponding unsaturated fatty alcohols with iodine values of 90 to 100.

2. Fatty compounds as claimed in claim 1, characterized in that the unsaturated fatty alcohols are subsequently alkoxylated in known manner.

3. Fatty compounds as claimed in claim 1, characterized in that the unsaturated fatty alcohols are subsequently alkoxylated, sulfated and neutralized in known manner.

4. Fatty compounds as claimed in claim 1, characterized in that the unsaturated fatty alcohols are subsequently sulfated and neutralized in known manner.

5. Fatty compounds as claimed in claim 1, characterized in that the unsaturated fatty alcohols are subsequently esterified in known manner with aliphatic carboxylic acids containing 1 to 22 carbon atoms and 0 and/or 1 to 3 double bonds.

6. A process for the production of unsaturated fatty compounds with improved low-temperature behavior, characterized in that
(a) new LS sunflower oil with an oleic acid content of more than 85% by weight and a stearic acid content of less than 3% by weight is transesterifed with methanol and
(b) the resulting methyl esters are hydrogenated in known manner to form the corresponding unsaturated fatty alcohols with iodine values of 90 to 100.

7. A process as claimed in claim 6, characterized in that the unsaturated fatty alcohols are subsequently alkoxylated in known manner.

8. A process as claimed in claim 6, characterized in that the unsaturated fatty alcohols are subsequently alkoxylated, sulfated and neutralized in known manner.

9. A process as claimed in claim 6, characterized in that the unsaturated fatty alcohols are subsequently sulfated and neutralized in known manner.

10. A process as claimed in claim 6, characterized in that the unsaturated fatty alcohols are subsequently esterified in known manner with aliphatic carboxylic acids containing 1 to 22 carbon atoms and 0 and/or 1 to 3 double bonds.

11. The use of the unsaturated fatty compounds claimed in claims 1 to 5 - either on their own or in the form of a mixture with known unsaturated fatty compounds - for the production of surface-active formulations.

12. The use of new LS sunflower oil with an oleic acid content of more than 85% by weight and a stearic acid content of less than 3% by weight for the production of unsaturated fatty alcohols with iodine values of 90 to 100 by transesterification and hydrogenation in accordance with claim 1.

## Revendications

1. Alcools gras insaturés dont l'indice d'iode est compris dans la plage de 90 à 100 et possédant un point de trouble de 1 °C, obtenables en
(a) transestérifiant avec du méthanol de la nouvelle huile de tournesol à faible teneur en acide stéarique, présentant une fraction d'acide oléique supérieure à 85 % en poids et une fraction d'acide stéarique inférieure à 3 % en poids et en
(b) hydrogénant les esters méthyliques résultants d'une manière connue en soi, en alcools gras insaturés correspondants, dont l'indice d'iode est compris dans la plage de 90 à 100.

2. Dérivés des alcools gras insaturés selon la revendication 1, **caractérisés en ce que** l'on alcoxyle ensuite les alcools gras insaturés d'une manière connue en soi.

3. Dérivés des alcools gras insaturés selon la revendication 1, **caractérisés en ce que** l'on alcoxyle, sulfate et neutralise ensuite les alcools gras insaturés d'une manière connue en soi.

4. Dérivés des alcools gras insaturés selon la revendication 1, **caractérisés en ce que** l'on sulfate et neutralise ensuite les alcools gras insaturés d'une manière connue en soi.

5. Dérivés des alcools gras insaturés selon la revendication 1, **caractérisés en ce que** l'on estérifie ensuite les alcools gras insaturés d'une manière connue en soi avec des acides carboxyliques aliphatiques comportant 1 à 22 atomes de carbone et 0 et/ou 1 à 3 doubles liaisons.

6. Procédé de production de matières grasses insaturées à comportement au froid amélioré, **caractérisé en ce que** l'on
(a) transestérifie avec du méthanol de la nouvelle huile de tournesol à faible teneur en acide stéarique, présentant une fraction d'acide oléique supérieure à 85 % en poids et une fraction d'acide stéarique inférieure à 3 % en poids et
(a) on hydrogène les esters méthyliques résultants d'une manière connue en soi en alcools gras insaturés correspondants dont l'indice d'iode est compris dans la plage de 90 à 100.

7. Procédé selon la revendication 6, **caractérisé en ce que** l'on alcoxyle ensuite les alcools gras insaturés d'une manière connue en soi.

8. Procédé selon la revendication 6, **caractérisé en ce que** l'on alcoxyle, sulfate et neutralise ensuite les alcools gras insaturés d'une manière connue en soi.

9. Procédé selon la revendication 6, **caractérisé en ce que** l'on sulfate et neutralise ensuite les alcools gras insaturés d'une manière connue en soi.

10. Procédé selon la revendication 6, **caractérisé en ce que** l'on estérifie ensuite les alcools gras insaturés d'une manière connue en soi avec des acides carboxyliques aliphatiques comportant 1 à 22 atomes de carbone et 0 et/ou 1 à 3 doubles liaisons.

11. Utilisation d'alcools gras et de leurs dérivés selon les revendications 1 à 5 - seuls ou en mélange avec des matières grasses insaturées connues - pour la production d'agents tensioactifs.

12. Utilisation de nouvelle huile de tournesol à faible teneur en acide stéarique, présentant une concentration supérieure à 85 % en poids d'acide oléique et inférieure à 3 % en poids d'acide stéarique pour la production d'alcools gras insaturés de la plage d'indice d'iode de 90 à 100, par transestérification et hydrogénation selon la revendication 6.
